# EUROPEAN PATENT APPLICATION

(11) **EP 4 620 468 A1**
(43) Date of publication of application: **24.09.2025**
(21) Application number: 23893814.6
(22) Date of filing: 21.11.2023
(51) Int. Cl.: A61K 31/41, A61K 47/40, A61K 9/08, A61K 9/19, A61P 35/00, A61P 35/04

(54) **BUTASELEN-CYCLODEXTRIN INCLUSION COMPLEX, PHARMACEUTICAL COMPOSITION, METHOD FOR PREPARING SAME, AND USE THEREOF**

(30) Priority: 21.11.2022 CN 202211459427
(71) Applicant: Shanghai Yuanxi Medicine Corp., Shanghai 201207 (CN)
(72) Inventor: ZENG, Huihui, Shanghai 201207 (CN); YIN, Hanwei, Shanghai 201207 (CN)
(74) Representative: ZBM Patents - Zea, Barlocci & Markvardsen
(86) International application number: PCT/CN2023/132850
(87) International publication number: WO 2024/109724

(57) **Abstract**

Disclosed are a butaselen-cyclodextrin inclusion complex, a pharmaceutical composition, a method for preparing same, and use thereof. The inclusion complex is a complex comprising butaselen or a derivative thereof and a cyclodextrin substance. The pharmaceutical composition comprises the inclusion complex and a pharmaceutically acceptable excipient. Formulations for injection comprising the cyclodextrin inclusion complex have good stability and safety, and exhibit good efficacy on brain tumor diseases or glioma.

## Description

### CROSS REFERENCE TO RELATED APPLICATIONS

This application claims the priority of a prior application No. 202211459427.9, filed to the CNIPA on November 21, 2022, entitled "BUTASELEN-CYCLODEXTRIN INCLUSION COMPLEX, PHARMACEUTICAL COMPOSITION, METHOD FOR PREPARING SAME, AND USE THEREOF". The entirety of the prior application is incorporated here by reference.

### TECHNICAL FIELD

The present disclosure belongs to the field of medicine, and relates to a butaselen-cyclodextrin inclusion complex, a pharmaceutical composition, a method for preparing the same, and use thereof.

### BACKGROUND

Pulmonary fibrosis is a type of changes in lung disease characterized by fibroblast proliferation and massive extracellular matrix (ECM) aggregation, accompanied by inflammatory injury and tissue structure destruction. Once it occurs, it cannot be reversed, and the impairment of the lung function will seriously affect the quality of life of patients and even endanger their lives.

A spinal cord belongs to the central nervous system, and its glioma incidence is low, accounting for 8-10% of all primary spinal cord tumors and 2-4% of all central nervous system tumors. According to their different cell sources, gliomas can be divided into ependymomas (60-70%) and various grades of astrocytomas (30-40%). Due to a limited transverse diameter of the spinal cord and the important physiological functions, the treatment is rather troublesome. At present, treatment guidelines for gliomas are mostly directed to intracranial diseases, and there is no good diagnosis and treatment standard for spinal glioma as a basis, resulting in great differences in treatment centers, thus affecting the quality of life of patients. At present, treatment regimens for spinal glioma are also diversified, including microsurgery, chemoradiotherapy, immunotherapy, and the like.

At present, there is a lack of significant and effective therapeutic drugs for pulmonary fibrosis and glioma in clinical practice internationally, especially for patients with non-IPF interstitial lung disease and fibrosis. The unmet clinical needs are extremely urgent.

Butaselen is a chemical small molecule drug designed to target thioredoxin reductase (TR). A series of completed studies have shown that butaselen exhibits a controlling effect on a whole chain of organ fibrotic diseases. By inhibiting inflammatory signals and key ECM synthesis pathways, butaselen can alleviate organ fibrosis manifestations and subsequent disease progression.

At present, butaselen tablets that have been approved for clinical use have therapeutic effects on pulmonary fibrotic diseases. However, due to the hepatic first-pass effect, the drug efficiency is reduced, and due to the influence of the blood-brain barrier, that is, no efficacy on intracranial glioma, there is an urgent need for more effective butaselen formulations for intracranial glioma.

According to the research and analysis of the properties of butaselen, butaselen is a poorly soluble drug, the solubility of butaselen in water is about 150 ng/mL after detection and analysis, and thus, its bioavailability in vivo is low, greatly limiting its clinical application. The following figure shows a chemical structural formula of butaselen.

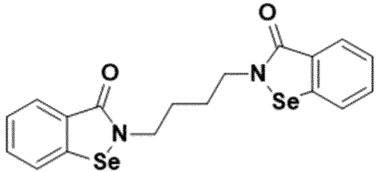

### SUMMARY

In order to improve the above technical problems, the present disclosure provides an inclusion complex, where the inclusion complex is a complex including butaselen or a derivative thereof and a cyclodextrin substance.

According to an embodiment of the present disclosure, the cyclodextrin substance is selected from a beta-cyclodextrin substance and/or a gamma-cyclodextrin substance, and for example, may be selected from one or two or more of hydroxypropyl-beta-cyclodextrin, sulfobutyl-beta-cyclodextrin, hydroxypropyl-gamma-cyclodextrin, methyl-beta-cyclodextrin and 2,6-dimethyl-beta-cyclodextrin, preferably hydroxypropyl-beta-cyclodextrin.

According to an embodiment of the present disclosure, the inclusion complex is formed by inclusion of butaselen or the derivative thereof by the cyclodextrin substance.

According to an embodiment of the present disclosure, a mass ratio of butaselen or the derivative thereof to the cyclodextrin substance is 1:(10-480), such as 1:(10-100), for example, 1:10, 1:20, 1:30, 1:40, 1:45, 1:49, 1:50 or 1: 60.

According to an embodiment of the present disclosure, an inclusion ratio of the cyclodextrin substance to butaselen or the derivative thereof is (1-15):1, e.g., (2-10):1, such as 1.5:1,2.5:1,3:1 or 4:1.

According to an embodiment of the present disclosure, the inclusion complex may be any one of the following inclusion complexes:
an inclusion complex formed by inclusion of butaselen by hydroxypropyl-beta-cyclodextrin, an inclusion complex formed by inclusion of butaselen by sulfobutyl-beta-cyclodextrin, an inclusion complex formed by inclusion of butaselen by hydroxypropyl-gamma-cyclodextrin, an inclusion complex formed by inclusion of butaselen methyl-beta-cyclodextrin, and an inclusion complex formed by inclusion of butaselen by 2,6-dimethyl-beta-cyclodextrin.

The present disclosure also provides a preparation method for the inclusion complex, including: inclusion of butaselen or a derivative thereof by using a cyclodextrin substance.

According to an embodiment of the present disclosure, selection of butaselen or the derivative thereof and the cyclodextrin substance and a mass ratio of butaselen or the derivative thereof to the cyclodextrin substance are as shown above.

According to an embodiment of the present disclosure, the inclusion is carried out in water or a Tween 80 solution.

According to an embodiment of the present disclosure, the water is water for injection.

According to an embodiment of the present disclosure, the Tween 80 solution is an aqueous Tween 80 solution. Preferably, the Tween 80 solution has a concentration of 0.01-0.1%, such as 0.05%.

According to an embodiment of the present disclosure, the preparation method includes the steps of:
1) first dissolving the cyclodextrin substance in the water or the Tween 80 solution to form a clear solution, then adding butaselen or the derivative thereof into the clear solution, and adding the water or the Tween 80 solution for quantification; and
2) after completion of the step 1), performing stirring and centrifugation, optionally filtering or not.

According to an embodiment of the present disclosure, a mass ratio of the cyclodextrin substance to the water in a system is (10-55):100, such as (20-50):100, for example, 30:100, 40:100 or 48:100; and a mass of the water in the system is the sum of the amount of the water used to dissolve the cyclodextrin substance and a mass of the water added for quantification.

According to an embodiment of the present disclosure, a mass ratio of the cyclodextrin substance to the Tween 80 solution in the system is (10-55):100, such as (20-50):100, for example, 30:100, 40:100 or 48:100; and a mass of the Tween 80 solution in the system is the sum of the amount of the Tween 80 solution used to dissolve the cyclodextrin substance and a mass of the Tween 80 solution added for quantification.

According to an embodiment of the present disclosure, the butaselen or the derivative thereof is added in the form of micro powder.

According to an embodiment of the present disclosure, the stirring is performed at a temperature of 25-80°C, preferably 40-70°C, such as 30°C, 40°C, 50°C, 60°C, or 70°C.

According to an embodiment of the present disclosure, the stirring is performed for 0.5-5 h, preferably 3 h.

According to an embodiment of the present disclosure, the centrifugation is performed at a rotational speed of 4000-10000 rpm, preferably 7000-9000 rpm, such as 9000 rpm.

According to an embodiment of the present disclosure, the centrifugation is performed for at least 5 min, such as 5-30 min, for example, 20 min.

According to an embodiment of the present disclosure, the preparation method further includes a step 3) of maintaining the clear solution obtained in the step 2) at a temperature of 2-8°C for 2-10 days, such as 3-8 days.

According to an embodiment of the present disclosure, the preparation method further includes a step 4) of filtering the solution obtained in the step 3) to obtain the inclusion complex.

According to an embodiment of the present disclosure, the filtering is microfiltering, for example, the solution is allowed to pass through a 0.45 micron filter.

The present disclosure also provides use of the inclusion complex in the preparation of a therapeutic pharmaceutical composition or formulation.

The present disclosure also provides a pharmaceutical composition or formulation, including the inclusion complex.

According to an embodiment of the present disclosure, the pharmaceutical composition or formulation may further include a pharmaceutically acceptable excipient, for example, the excipient includes, but is not limited to, one or more of a vehicle, a lubricant, a binder, a disintegrant, a solvent, a dissolution aid, a suspending agent, an isotonic agent, a pH adjusting agent, a buffer, a preservative, an antioxidant, a coloring agent, a foaming agent and a flavoring agent and the like, preferably one or more of the solvent, the isotonic agent, and the pH adjusting agent. In some embodiments, the pharmaceutical composition or formulation contains NaCl.

In one embodiment, the pharmaceutical composition or formulation includes the following raw materials: butaselen or a derivative thereof, a cyclodextrin substance, water for injection, Tween, and a pH adjusting agent.

In another embodiment, the pharmaceutical composition or formulation includes the following raw materials: butaselen or a derivative thereof, a cyclodextrin substance, water for injection, and a pH adjusting agent.

Preferably, the mass percentage of butaselen or the derivative thereof in the pharmaceutical composition or formulation is 0.4-5%, such as 1-4%.

Preferably, the mass percentage of the cyclodextrin substance in the pharmaceutical composition or formulation is 30-50%, such as 35-45%.

According to an embodiment of the present disclosure, the formulation is administered by injection, such as intrathecal injection, intraperitoneal injection, subcutaneous injection, intravenous injection, acupoint injection, intramuscular injection, etc.

According to an embodiment of the present disclosure, the formulation may be an injection, such as an injection solution or a lyophilized powder for injection.

According to an embodiment of the present disclosure, the injection solution can be prepared by filtering and sterilizing the inclusion complex in the form of a solution prepared as described above. Preferably, the filtering is microfiltering, for example, the solution is allowed to pass through a 0.22 micron filter.

According to an embodiment of the present disclosure, the lyophilized powder for injection may be prepared by filtering, sterilizing, and aseptically lyophilizing the inclusion complex in the form of a solution prepared as described above. Preferably, the filtering is microfiltering, for example, the solution is allowed to pass through a 0.22 micron filter.

According to an embodiment of the present disclosure, the pharmaceutical composition or formulation contains a second active substance, for example, the second active substance is an anti-tumour drug, preferably a glioma therapeutic agent, for example, temozolomide (TMZ).

In some embodiments, a mass ratio of butaselen to the second active material is (2-20):1, e.g., 3:1, 4:1, 5:1, 6:1, 7:1, 8:1, 9:1, 10:1, 12:1 or 15:1.

The present disclosure also provides use of the pharmaceutical composition or formulation in the prevention and/or treatment of at least one of the following diseases: brain tumor disease or glioma, including but not limited to one or more of intracranial glioma (i.e., brain glioma), spinal glioma (e.g., spinal astrocytoma, preferably high-grade spinal astrocytoma), medulloblastoma, and brain tumor formed from metastatic brain tumor, and the like.

### Beneficial effects of the present disclosure:

The present disclosure provides a cyclodextrin inclusion complex of butaselen and a derivative thereof, which greatly increases the solubility of poorly soluble butaselen, and the preparation process is simple and easy to industrialize.

An injection containing the cyclodextrin inclusion complex has good stability, safety, and good therapeutic effects on pulmonary fibrosis disease and glioma.

Compared with oral administration, the injection can still achieve good therapeutic effects on brain glioma treatment at a low dose; and the injection has also been found to enhance drug distribution results of an active ingredient into the brain, and has an important value for improving brain tissue distribution of a drug.

The inventors have also unexpectedly discovered that the injection of the cyclodextrin inclusion complex containing butaselen and the derivative thereof can be used in combination with TMZ to treat brain glioma, effectively reducing the dose of TMZ administered, slowing the toxic and side effects of the drug on treated subjects, and improving the safety of the drug.

At the same time, the injection also has a good therapeutic effect on high-grade spinal astrocytoma.

### BRIEF DESCRIPTION OF THE DRAWINGS

FIG. 1 shows comparison of the efficacy of oral administration and intraperitoneal injection of a BS single drug (a red box) and BS+TMZ (a blue box).
FIG. 2 shows the body weight and tumor changes of a U87 glioma model in nude mice treated with BS, TMZ, and a combination of BS and TMZ. (A) Body weight of mice in each group during the experiment; (B) Tumor weight of mice in each group at the end of the experiment; (C) Tumor volume of mice in each group during the experiment; and (D) Photographs of tumors in mice in each group at the experimental endpoint. Data in (A), (B), and (C) were presented as mean±SD, n=5 or 6. * indicates that there is a statistical difference compared with a control group, *P<0.05, **P<0.01.
FIG. 3 is a tumor volume change curve of NPG mice during treatment (n=8).
FIG. 4 is a bar graph of a body weight change rate of NPG mice after administration.
FIG. 5 is a graph showing mean tumor volume of NPG mice on D24.

### DETAILED DESCRIPTION

In the following, the technical solution of the present disclosure will be described in further detail with reference to specific examples. It should be understood that the following examples only illustrate and explain the present disclosure and are not to be construed as limiting the protection scope of the present disclosure. All technologies realized based on the above contents of the present disclosure are covered in the scope of protection of the present disclosure.

Unless otherwise specified, raw materials and reagents used in the following examples are commercially available or can be prepared by known methods.

### Example 1

2,6-Dimethyl-beta-cyclodextrin was dissolved in approximately 51.9 g of water (cyclodextrin:water = 55:100) under magnetic stirring. 100 mg of Butaselen was added to the clear pale yellow solution under magnetic stirring, and a final weight was made up to 100 g with water. Heating was performed in a water bath of 70°C while stirring for 3 h, and the obtained solution was allowed to pass through a 0.45 micron filter to obtain a clear pale yellow solution. It was found that the butaselen concentration was 1869.30 µg/g by HPLC analysis.

### Example 2

4.8 g of Hydroxypropyl-beta-cyclodextrin was dissolved in approximately 4.7 g of a 0.05% Tween 80 solution under magnetic stirring. 480 mg of Butaselen was added to the clear colorless solution under magnetic stirring, and a final weight was made up to 10 g with a 0.05% Tween 80 solution. The obtained solution was stirred for 2 h, and centrifuged at 10000 rpm for 10 min, and the solution was allowed to pass through a 0.45 micron filter to obtain a clear colorless solution. It was found that the butaselen concentration was 560 µg/g by HPLC analysis.

The solution thus obtained was kept at 4°C for 8 days. A white precipitate was formed under these conditions, and was removed from the solution by filtration through a 0.45 micron filter.

A clear colorless solution was obtained, and it was found that the butaselen concentration was 430 µg/g by HPLC analysis.

### Example 3

9.6 g of Hydroxypropyl-beta-cyclodextrin was dissolved in approximately 9.4 g of a 0.05% Tween 80 solution under magnetic stirring. 196 mg of Butaselen was added to the clear colorless solution under magnetic stirring, and a final weight was made up to 20 g with a 0.05% Tween 80 solution. The obtained solution was stirred at 70°C for 5 h, and centrifuged at 9000 rpm for 10 min, and the solution was allowed to pass through a 0.45 micron filter to obtain a clear colorless solution. It was found that the butaselen concentration was 414 µg/g by HPLC analysis.

The solution thus obtained was kept at 4°C for 3-8 days. A white precipitate was formed under these conditions, and was removed from the solution by filtration through a 0.45 micron filter.

A clear colorless solution was obtained, and it was found that the butaselen concentration was 352 µg/g by HPLC analysis.

### Example 4

9.6 g of Hydroxypropyl-beta-cyclodextrin was dissolved in approximately 9.4 g of water for injection under magnetic stirring. 192 mg of Butaselen was added to the clear colorless solution under magnetic stirring, and a final weight was made up to 20 g with water. The obtained solution was stirred at 40°C for 3 h and centrifuged at 9000 rpm for 10 min. The solution was allowed to pass through a 0.45 micron filter to obtain a clear colorless solution. It was found that the butaselen concentration was 366 µg/g by HPLC analysis.

The solution thus obtained was kept at 4°C for 3-8 days. A white precipitate was formed under these conditions, and was removed from the solution by filtration through a 0.45 micron filter.

A clear colorless solution was obtained, and it was found that the butaselen concentration was 310 µg/g by HPLC analysis.

### Example 5

4.8 g of Sulfobutyl-beta-cyclodextrin was dissolved in approximately 4.7 g of water for injection under magnetic stirring. 480 mg of Butaselen was added to the clear colorless solution under magnetic stirring, and a final weight was made up to 10 g with water. The obtained solution was stirred at 25°C for 1 h and centrifuged at 10000 rpm for 10 min. The solution was allowed to pass through a 0.45 micron filter to obtain a clear colorless solution. It was found that the butaselen concentration was 133 µg/g by HPLC analysis.

### Example 6

9.6 g of Hydroxypropyl-beta-cyclodextrin was dissolved in approximately 9.4 g of an aqueous solution for injection under magnetic stirring. 196 mg of Butaselen was added to the clear colorless solution under magnetic stirring, and a final weight was made up to 20 g with water. The obtained solution was stirred at 70°C for 3 h and centrifuged at 9000 rpm for 10 min. The solution was allowed to pass through a 0.45 micron filter to obtain a clear colorless solution. It was found that the butaselen concentration was 601 µg/g by HPLC analysis.

The solution thus obtained was kept at 4°C for 3-8 days. A white precipitate was formed under these conditions, and was removed from the solution by filtration through a 0.45 micron filter.

A clear colorless solution was obtained, and it was found that the butaselen concentration was 473 µg/g by HPLC analysis.

BS in the following Experimental examples 1-3 is an abbreviation for butaselen;
In injection groups containing BS of Experimental examples 1-3, BS is present in the form of a cyclodextrin inclusion complex. Taking a BS group of Experimental example 1 as an example, a formula includes the following raw materials: an inclusion complex formed from butaselen and hydroxypropyl-gamma-cyclodextrin at a mass ratio of 1:40 and a Tween 80 solution having a concentration of 0.05%; and other groups were prepared with reference to the BS group of Experimental example 1.

### Experimental example 1

### 1. Test sample

BS and TMZ solution (for gavage administration of a U87 brain glioma subcutaneous model in Balb/c nude mice)

| Group | TMZ mass (mg) | BS Mass (mg) | Solvent and volume |
|---|---|---|---|
| Control group | 0 | 0 | 0.5% CMC-Na, 5 mL |
| TMZ Group | 30 | 0 | 0.5% CMC-Na, 5 mL |
| BSL group | 0 | 10 | 0.5% CMC-Na, 5 mL |
| BSM group | 0 | 90 | 0.5% CMC-Na, 5 mL |
| BSH group | 0 | 180 | 0.5% CMC-Na, 5 mL |
| TMZ+BSM group | 30 | 90 | 0.5% CMC-Na, 5 mL |

After preparation of a solution or a suspension, a dose administered to mice was 5 mL/kg.

BS and TMZ solution (for intraperitoneal administration of a U87 brain glioma subcutaneous model in Balb/c nude mice)

| Group | TMZ mass (mg) | BS Mass (mg) | Solvent and volume |
|---|---|---|---|
| Control group | 0 | 0 | 0.9% NaCl, 5 mL |
| TMZ Group | 10 | 0 | 0.9% NaCl, 5 mL |
| BS Group | 0 | 30 | 0.9% NaCl, 5 mL |
| TMZ+BS treatment group | 10 | 90 | 0.5% CMC-Na, 5 mL |
| TMZ+BS prevention group | 105 | 90 | 0.5% CMC-Na, 5 mL |

After preparation of a solution or a suspension, a dose administered to mice was 5 mL/kg.

### 2. Experimental methods

### 2.1 Gavage administration experiment of U87 brain glioma subcutaneous model in Balb/c nude mice

After 2 days of animal acclimation, qualified animals were retained and randomized into 6 groups, with 7 animals in each group. Mice were inoculated subcutaneously in right axillas with U87 cells at 2×10⁶/mouse. Administration was started to be performed 12 days after inoculation, which was day 0 of treatment. Administration was performed for a total of 11 days.

A drug treatment regimen was as follows: Blank control group (0.5% CMC-Na, i.g., q.d.); TMZ group (30 mg/kg, i.g., q.d.); BSL group (10 mg/kg, i.g., q.d.); BSM group (90 mg/kg, i.g., q.d.); BSH group (180 mg/kg, i.g., q.d.); and BSM+TMZ group (30 mg/kg of TMZ, 90 mg/kg of BS, i.g., q.d.).

### 2.2 Administration by injection (intraperitoneal) experiment of U87 brain glioma subcutaneous model in Balb/c nude mice

After 2 days of animal acclimation, qualified animals were retained and randomized into 5 groups, with 6 animals in each group of a control group, a TMZ group, and a BS group. There were 5 animals in each group of the BS+TMZ treatment group and the BS+TMZ prevention group. Mice in the BS group were inoculated subcutaneously in right axillas with U87 cells at 2×10⁶/mouse. Administration was started to be performed 14 days after inoculation, which was day 0 of treatment. Administration was performed for a total of 18 days.

A drug treatment regimen was as follows: Blank control group (0.9% NaCl, i.p., q.d.); TMZ group (10 mg/kg, i.p., qd.); BS group (30 mg/kg, i.p., q.d.); BS+TMZ treatment group (10 mg/kg of TMZ, i.p., qd., and 90 mg/kg of BS, i.g., q.d.); BS+TMZ prevention group (10 mg/kg of TMZ, i.p., qd., and 90 mg/kg of BS, i.g., q.d.). TMZ cells were injected into tail veins.

The general condition of the animals and the time of tumor occurrence were recorded daily during the experiment, a long diameter and a short diameter of tumors in the mice were recorded daily, and a tumor volume was calculated according to a formula: long diameter×(short diameter)²×0.5236, and a body weight of the animals were measured every 3 days. After 18 days of administration, anesthesia was performed with chloral hydrate, and eyeballs were removed for blood collection. The mice were sacrificed, tumors, hearts, livers, spleens, lungs, kidneys, thymuses, whole brains, and intestines were rapidly isolated, blood stains were washed away with ice saline, the tumors, hearts, livers, spleens, lungs, kidneys, thymuses, whole brains, and intestines were wiped to be dry with filter paper, whether there were lesions in internal organs of the animals were observed, and the tumors, hearts, livers, spleens, lungs, kidneys, thymuses, whole brains, and intestines were weighed. A portion of tumor tissue was taken and stored in liquid nitrogen for RNA and protein extraction. Another portion of tumor tissue was placed in a tissue fixative solution for fixation. Distribution of the Se content in brain tissue was detected by Inductively coupled plasma-Mass Spectrometry (ICP-MS).

### 3. Experimental results

### 3.1 Comparison of the efficacy of BS gavage and injection on U87 glioma model in nude mice

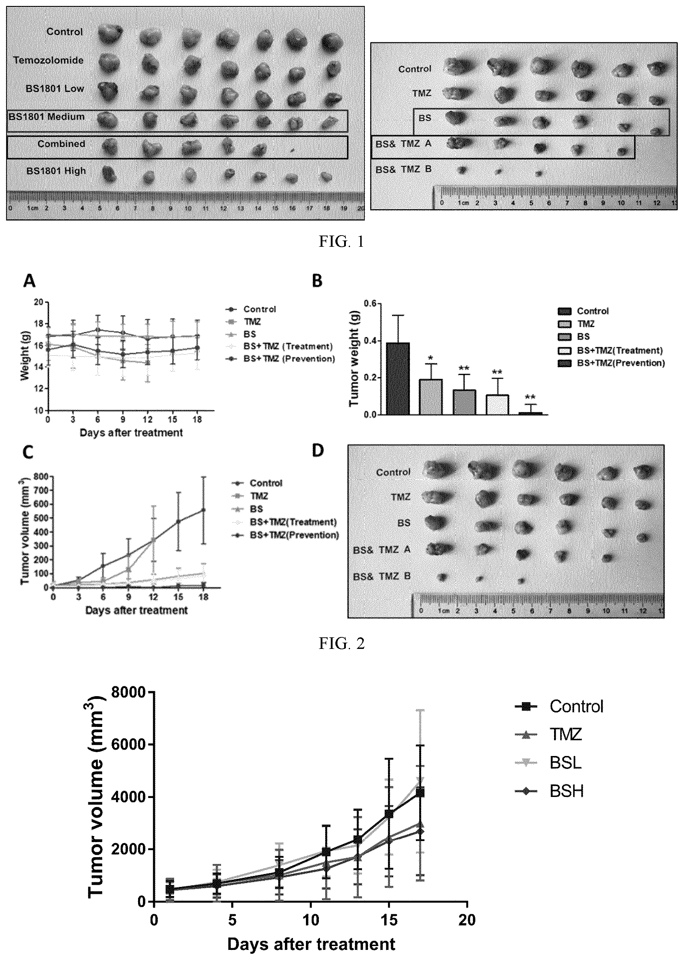

The efficacy of BS single drug gavage/injection (intraperitoneal) was compared, as shown in FIG. 1 and Table 1. By comparing the efficacy of BS administrated through gavage (90 mg/kg) and intraperitoneal injection (30 mg/kg), it was found that a tumor volume inhibition rate of BS administrated through gavage (90 mg/kg) was 78.87% and a tumor volume inhibition rate of BS administrated through intraperitoneal injection (30 mg/kg) was 81.12%. The results indicates that the efficacy of inhibiting tumor growth was more obvious by intraperitoneal injection on the premise of reducing the dosage of BS. It was also shown that BS single drug injection could significantly inhibit the proliferation of human-derived U87 glioma cells, and the inhibitory effect was far better than that of TMZ.

**Table 1 Comparison of efficacy of BS single drug and BS+TMZ administrated through oral administration/intraperitoneal injection.**

| Dosing regimen | Administration dosage (mg/kg) | Mode of administration | Tumor volume (mm³) | Volume inhibition rate (%) |
|---|---|---|---|---|
| BS | 90 | Oral administration | 122.05±33.15 | 78.87 |
| | 30 | Intraperitoneal injection | 105.18±66.62 | 81.12 |
| BS&TMZ | 90+30 | Oral administration | 124.32±93.42 | 78.48 |
| | 90+10 | Intraperitoneal injection | 87.53±75.70 | 84.29 |

By comparing the efficacy of the combined use of BS+TMZ administrated through gavage/intraperitoneal injection, a tumor volume inhibition rate of BS+TMZ administrated through oral administration (90+30 mg/kg) was 78.48% and a tumor volume inhibition rate of BS+TMZ administrated through intraperitoneal injection (90+10 mg/kg) was 84.29%. The efficacy of inhibiting tumor growth was more obvious by intraperitoneal injection on the premise of reducing the dosage of TMZ. It was suggested that the BS single drug and the combined use of BS+TMZ administrated through intraperitoneal injection had more obvious efficacy in inhibiting tumor growth compared with oral administration, and the dosage of BS or TMZ drug could be reduced and the toxicity of TMZ could be reduced.

### 3.2 Comparison of distribution of BS administrated through gavage and intraperitoneal injection in brain tissue of U87 glioma model in nude mice

Brain tissues of the mice after the experiment of BS+TMZ gavage (90+10 mg/kg) and BS intraperitoneal injection (30 mg/kg) were taken and detected by ICP-MS. By detecting the selenium content per gram of brain tissue, the BS content per gram of brain tissue was calculated. The distribution of BS in brain tissue is shown in Table 2, and BS is distributed in brain tissue. The BS content of the brain tissue after BS was administered through intraperitoneal injection at a dose of 30 mg/kg was significantly higher than that after BS was administered through gavage at a dose of 90 mg/kg. It is suggested that the administration of BS by intraperitoneal injection facilitates the entry of BS into the brain tissue, achieving the effect of BS dosage reduction.

**Table 2 Distribution of BS in brain tissue of U87 nude mice after administration**

| Dosing regimen | Administration dosage (mg/kg) | Mode of administration | BS (ng/g) |
|---|---|---|---|
| BS | 30 | Injection dosage form (intraperitoneal) | 120.61 |
| BS+TMZ | 90+10 | Gavage | 38.46 |

By comparing the efficacy of BS gavage and injection (intraperitoneal) on the U87 glioma model in the nude mice, it was found that the BS single drug and the combined use of BS+TMZ administrated through injection (intraperitoneal) had more obvious efficacy in inhibiting tumor growth compared with oral gavage administration, and the dosage of BS or TMZ drug could be reduced and the toxicity of TMZ could be reduced. Through detection of the distribution of BS in brain tissue by ICP-MS, it was found that the administration of BS by intraperitoneal injection was more beneficial to the entry of BS into the brain tissue, achieving the effect of BS dosage reduction.

### Experimental example 2

### Pharmacodynamic study of injection of BS in combination with TMZ into U87 glioma model in nude mice

### 1. BS and TMZ synergistically inhibit tumor proliferation in U87 tumor-bearing nude mice.

Female six-week-old Balb/c Nude mice were selected to establish an animal model and inoculated subcutaneously with U87 human-derived glioma cells at 2×10⁶/mouse. Administration was started to be performed on day 14 after inoculation, and was continued to be performed for 18 days.

To compare the difference in efficacy between oral gavage administration and intraperitoneal injection administration, the control group, the TMZ group (10 mg/Kg) and the BS group (30 mg/Kg) were administered intraperitoneally once a day in this round of experiment. The mice in the control group were given 0.9% NaCl.

To compare the therapeutic efficacy and preventive efficacy of the combined application of BS and TMZ, combined administration groups were set in this round of experiment: a BS+TMZ treatment group (90 mg/Kg of BS&10 mg/Kg of TMZ) and a BS+TMZ prevention group (90 mg/Kg of B S& 10 mg/Kg of TMZ). The B S+TMZ treatment group was abbreviated as a BS+TMZ (A) group, and tumors with a volume of more than 20 mm³ were normally formed in the mice on day 14 after U87 inoculation, and the administration was started to be performed to observe the efficacy of the combined use on inhibiting tumor proliferation. The BS+TMZ prevention group is abbreviated as a BS+TMZ (B) group, and tumors with a volume of less than 20 mm³ were formed in the mice on day 14 after U87 inoculation, and the administration was started to observe the efficacy of the combined use on the prevention of tumor formation. The mice in the combined groups were administered by single daily oral gavage. The start time of administration was Day 1, a second day of administration was Day 2, and so on.

The body weight changes of the mice in each group during administration are shown in FIG. 2 (A). The body weight of the mice in the TMZ group (30 mg/Kg) was significantly decreased, and the experiment in the TMZ group was ended on day 12 of administration due to the extremely poor animal status. The mice in the BS group, the BS+TMZ (A) group, and the BS+TMZ (B) group had normal diet and water intake, normal activities, good mental status, and smooth and shiny hair, suggesting that BS had no obvious toxic and side effects on tumor-bearing nude mice.

During the experiment, the tumor volume changes of the mice in each group are as shown in FIG. 2 (C), and the tumor volumes of the mice in the BS group, the BS+TMZ (A) group, and the BS+TMZ (B) group were significantly lower than those of the mice in the control group (p<0.01). After the end of the experiment, photographs of the tumors of the mice in each group are shown in FIG. 2 (D), and it can be seen that the tumors of the mice in the administration groups are significantly reduced compared with the control group. The results of statistical analysis of weighing of tumors are shown in FIG. 2 (B) and Table 3.

**Table 3 Tumor volume and weight of mice in each group at the end of the experiment**

| Group | Dose (mg/kg) | Tumor volume (mm³) | Volume inhibition rate (%) | Tumor weight (g) | Weight inhibition rate (%) |
|---|---|---|---|---|---|
| Control | 0 | 557.09±241.73 | -- | 0.39±0.15 | -- |
| TMZ | 10 | 343.23±245.20 | -- | 0.19±0.08* | -- |
| BS | 30 | 105.18±66.62** | 81.12 | 0.13±0.08** | 66.67 |
| BS+TMZ (A) | 90+10 | 87.53±75.70** | 84.29 | 0.11±0.09** | 71.79 |
| BS+TMZ (B) | 90+10 | 19.74±16.94*** | 96.46 | 0.01±0.04** | 97.44 |

Data are expressed as mean±SD, n=5 or 6. Due to the extremely poor conditions of the mice in the TMZ group on day 12 of administration, the experiment was ended early, so data was missing. * indicates that there is a significant difference compared with the control group, *p<0.05; **p<0.05, and ***p<0.001.

After analysis of the pharmacodynamic performance of the U87 glioma model in the nude mice administered intraperitoneally in the control group, the TMZ group, and the BS group, it was found that the tumor volume and tumor weight in the BS group (30 mg/Kg) were significantly lower than those in the control group, which could significantly inhibit tumor growth. By the end of the experiment, the tumor volume inhibition rate and weight inhibition rate of the mice in the BS group were 81.12% and 66.67%, respectively (Table 3). This result suggests that a reduced dose of BS can also significantly inhibit tumor growth when BS is administered by intraperitoneal injection. Due to the extremely poor condition of the mice in the TMZ group on day 12 of administration, the experiment was ended early, so data was not compared.

The distribution of BS in mouse brain tissue was examined as follows:
Brain glioma is located in the skull and has a special lesion location. Thus, it is critical whether a drug can cross the blood-brain barrier and act at the lesion site. In order to detect whether BS can reach brain tissue through the blood-brain barrier, female six-week-old ICR mice were selected for the study. The mice were intraperitoneally injected with 90 mg/kg of BS, and brain tissues before administration and brain tissues at 0.5 h, 1 h, 2 h and 3 h after administration were taken for ICP-MS detection. The BS content per gram of brain tissue was calculated by detecting the selenium content per gram of brain tissue.

The distribution of BS in brain tissue is shown in Table 4, and BS is distributed in the brain tissue. The BS content in the brain tissue reached 438.75 ng/g at 0.5 h after administration, and the BS content in the brain tissue reached a peak of 528.50 ng/g at about 2 h of administration and then decreased. This result indicates that BS can break through the blood-brain barrier, and be distributed in mouse brain tissue.

**Table 4 Distribution of BS in brain tissue after intraperitoneal injection of 90 mg/kg of BS in ICR mice**

| | | | | |
|---|---|---|---|---|
| Treatment time (h) | 0.5 | 1 | 2 | 3 |
| BS (ng/g) | 438.75 | 371.80 | 528.50 | 418.81 |

Experimental example 3 Pharmacodynamic study of BS injection on subcutaneous patient-derived xenograft (PDX) mouse model of large-volume high-grade astrocytoma in human spinal cord

### 1. Method:

1) Grouping of animals: the animals were subcutaneously inoculated with tumor tissue pieces, and after tumors were formed in the animals, the animals were grouped based on a principle of a similar mean tumor volume;
2) Mode of administration: intraperitoneal injection of BS and TMZ (i.p., q.d.×17 days);
3) Detection indexes: mouse body weight and subcutaneous tumor volume.

### 2. Test sample preparation

BS injections, with a drug content being 30 mg of BS and 90 mg of BS; and 1 mg of TMZ was weighed and dissolved in 1 ml of 0.5% CMC-Na and the obtained solution was uniformly dispersed by an ultrasound-assisted manner to obtain a TMZ drug solution, which was prepared immediately before use each time.

### 3. Experimental animals

Animal species: NPG mice, Animal grade: SPF grade, and Sex and number: female, 32
Animal weight range: 20-25 g, Animal age range: 5-6 weeks old, and Animal source: Beijing Vitalstar Biotechnology Co., Ltd.

### 4. Animal screening and grouping

The animals were randomly divided into four groups according to Table 5, with 8 animals in each group of a control group, a BSL group, a BSH group and a TMZ group.

**Table 5**

| Group | Administration dosage (mg/kg each time) | Administration concentration (mg/mL) | Administration capacity (mL/kg) |
|---|---|---|---|
| Control | 0 | 0 | 5 |
| TMZ | 5 | 0.5 | 5 |
| BSL | 30 | 3 | 5 |
| BSH | 90 | 9 | 5 |

### 5. Mode and frequency of administration

Tumor masses (2-3 mm³) of high-grade astrocytoma in a human spinal cord were prepared and inoculated in right axillas of NPG mice, and after inoculation, a mouse status was routinely observed and the tumor size was measured. Screening was performed according to the tumor volume, animals with a tumor volume of 450 mm³ were selected for the experiment, and those with an excessive tumor volume and no tumor formation were not selected. A total of 32 suitable tumor-bearing animals were screened for the experiment, and were divided into 4 experimental groups, with 8 mice in each group. The day of first administration was recorded as D1, a second day of administration was recorded as D2, and so on. All animals were administered through single daily intraperitoneal injection for 24 consecutive days.

### 6. Dosage design basis

The results of previous experiments showed that the BS injection was administered by gavage in a dose range of 90, 180 and 360mg/kg, and there was no toxic reaction in animals, and the tumor inhibitory effect was dose-dependent. Thus, a dose of 90 mg/kg was selected as a maximum dose for intraperitoneal injection in this experiment using this injection dosage form.

### 7. Detection indexes

### 7.1 Body weight

All animals were observed once a day during the experiment period. The observation content included death or dying, feces, a feeding status, a mental status, and behavioral activities, etc. If any abnormal situation was found, it was recorded in time. All animals were weighed once before grouping, three times per week after administration, and once before euthanasia.

### 7.2 Tumor volume

During the experiment, a long diameter and a short diameter of the tumors in all the animals were measured daily. And a tumor volume (mm³) was calculated and a formula was as follows: Tumor volume=0.5236×long diameter×short diameter×short diameter.

### 8. Experimental results

### 8.1. Tumor growth curve

In this experiment, the experiment was terminated when the mice in the experimental groups had moribund vital signs or had a significant decrease in body weight (>20%). No mice died in each experimental group at the end of the experiment, and experimental data of a total of 32 mice was included in the statistics. FIG. 3 is a tumor volume change curve of NPG mice after grouping during treatment with BS and TMZ.

### 8.2. Body weight

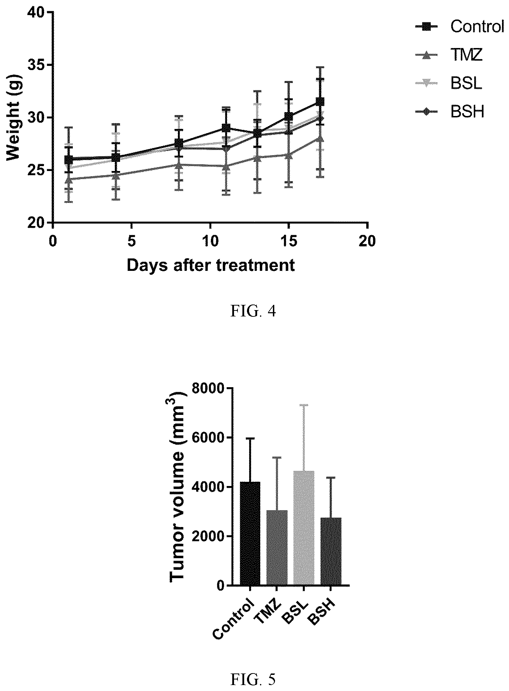

No clinical abnormal symptoms or animal deaths due to drug toxicity were observed during this experiment. FIG. 4 shows the change in body weight of mice in each group during administration. The BS group (30 and 90 mg/kg) showed no significant weight loss at this dose studied, suggesting that intraperitoneal injection of BS had no obvious toxic and side effects on the NPG mice. During the administration, the mice had a good mental state, indicating low drug toxicity, which is beneficial for long-term maintenance.

### 8.3. Mouse subcutaneous tumor volume on the day of dissection

As shown in FIG. 5, BS showed significant tumor inhibition in the 90 mg/kg dose group and the TMZ group. At the end of the experiment, tumor sizes in the Control group, the BS groups (30 and 90 mg/kg) and the TMZ group were 4155.70±1809.90, 4593.83±2725.69, 2999.76±2191.03, and 2693.29±1682.23, respectively. At the end of the experiment, the tumor volume inhibition rates in the low-dose BS group and the high-dose BS group were -10.54% and 35.19%, respectively, and the tumor volume inhibition rate in the TMZ group (5 mg/kg) was 27.82%, indicating that BS (90 mg/kg) and TMZ could inhibit spinal glioma growth.

Embodiments of the present disclosure have been described above. However, the present disclosure is not limited to the embodiments described above. Any modifications, equivalent replacements, improvements and the like made without departing from the spirit and scope of the present disclosure should be included within the scope of protection of the present disclosure.

## Claims

1. An inclusion complex, wherein the inclusion complex is a complex comprising butaselen or a derivative thereof and a cyclodextrin substance.

2. The inclusion complex according to claim 1, wherein the cyclodextrin substance is selected from a beta-cyclodextrin substance and/or a gamma-cyclodextrin substance, and for example, can be selected from one or two or more of hydroxypropyl-beta-cyclodextrin, sulfobutyl-beta-cyclodextrin, hydroxypropyl-gamma-cyclodextrin, methyl-beta-cyclodextrin and 2,6-dimethyl-beta-cyclodextrin;
preferably, the inclusion complex is formed by inclusion of butaselen or the derivative thereof by the cyclodextrin substance; and
preferably, a mass ratio of butaselen or the derivative thereof to the cyclodextrin substance is 1:(10-480), for example, 1:(10-100).

3. A preparation method for the inclusion complex according to claim 1 or 2, comprising inclusion of butaselen or the derivative thereof by using the cyclodextrin substance.

4. The preparation method according to claim 3, wherein the inclusion is carried out in water or a Tween 80 solution.

5. The preparation method according to claim 3 or 4, comprising the steps of:
1) first dissolving the cyclodextrin substance in the water or the Tween 80 solution to form a clear solution, then adding butaselen or the derivative thereof into the clear solution, and adding the water or the Tween 80 solution for quantification; and
2) after completion of the step 1), performing stirring and centrifugation, optionally filtering or not, wherein
preferably, a mass ratio of the cyclodextrin substance to the water in a system is (10-55): 100, and a mass of the water in the system is the sum of the amount of the water used to dissolve the cyclodextrin substance and a mass of the water added for quantification; and
preferably, a mass ratio of the cyclodextrin substance to the Tween 80 solution in the system is (10-55):100, and a mass of the Tween 80 solution in the system is the sum of the amount of the Tween 80 solution used to dissolve the cyclodextrin substance and a mass of the Tween 80 solution added for quantification.

6. Use of the inclusion complex according to claim 1 or 2 in the preparation of a therapeutic pharmaceutical composition or formulation.

7. A pharmaceutical composition or formulation, comprising the inclusion complex according to claim 1 or 2.

8. The pharmaceutical composition or formulation according to claim 7, further comprising a pharmaceutically acceptable excipient, wherein
preferably, the formulation is administered by injection; and
preferably, the formulation is an injection, such as an injection solution or a lyophilized powder for injection.

9. The pharmaceutical composition or formulation according to claim 7 or 8, comprising a second active substance, for example, the second active substance being an anti-tumour drug, preferably a glioma therapeutic agent, for example, temozolomide (TMZ).

10. Use of the pharmaceutical composition or formulation according to any one of claims 7 to 9 in the prevention and/or treatment of at least one of the following diseases: brain tumor disease or glioma, comprising but not limited to one or more of intracranial glioma (i.e., brain glioma), spinal glioma (e.g., spinal astrocytoma, preferably high-grade spinal astrocytoma), medulloblastoma, and brain tumor formed from metastatic brain tumor.
